# EUROPEAN PATENT APPLICATION

(11) **EP 4 534 089 A1**
(43) Date of publication of application: **09.04.2025**
(21) Application number: 23811176.9
(22) Date of filing: 26.05.2023
(51) Int. Cl.: A61K 31/53, A61P 31/14, A61P 29/00, A61P 11/14, A61P 11/04, A61P 11/00

(54) **PHARMACEUTICAL COMPOSITION OF NUCLEOSIDE-DERIVED COMPOUND, PREPARATION METHOD THEREFOR, AND USE THEREOF**

(30) Priority: 27.05.2022 CN 202210587803
(71) Applicant: Shenzhen Antiv Pharma Co., Ltd., Shenzhen, Guangdong 518000 (CN)
(72) Inventor: LI, Shuo, Shenzhen, Guangdong 518000 (CN); LI, Guanguan, Shenzhen, Guangdong 518000 (CN); LIU, Xinjun, Shenzhen, Guangdong 518000 (CN)
(74) Representative: Zaboliene, Reda
(86) International application number: PCT/CN2023/096521
(87) International publication number: WO 2023/227106

(57) **Abstract**

The present invention provides a pharmaceutical composition of a nucleoside-derived compound, a preparation method therefor, and use thereof, and belongs to the field of medicines. The pharmaceutical composition comprises a compound SHEN26 and a pharmaceutically acceptable excipient. The pharmaceutically acceptable excipient comprises at least one selected from a diluent, a disintegrant, an adhesive, a lubricant, and a glidant. The pharmaceutical composition has the advantages of a high dissolution rate, good compatibility of raw and auxiliary materials, good stability, high bioavailability, and the like.

## Description

### Technical Field

The present invention relates to the field of pharmaceutical formulations, in particular to a pharmaceutical composition of a nucleoside derivative compound, a preparation method therefor, and use thereof.

### Background

The novel coronavirus is an enveloped single-stranded RNA virus belonging to the β-genus coronavirus. Similar to SARS and MERS, the SARS-CoV-2 genome encodes non-structural proteins: 3-chymotrypsin-like protease (3CLpro), papain-like protease (PLpro), helicase, and RNA-dependent RNA polymerase (RdRp); structural proteins: such as spike glycoprotein and accessory proteins. The surface spike glycoprotein of the novel coronavirus binds to the angiotensin-converting enzyme (ACE2) receptor on the surface of human cells, thereby infecting the epithelial cells of the human respiratory tract. After the virus enters the host cell, it disassembles and releases the nucleocapsid and viral RNA into the cytoplasm. The 5' end open reading frame (ORF1a/b) of the viral RNA encodes polyproteins (ppla and pplab), which play a crucial role in the processing and maturation of enzymes required for viral replication. ppla and pplab can be cleaved by papain-like protease (PLpro) and 3-chymotrypsin-like protease (3CLpro) to produce non-structural proteins, including RNA-dependent RNA polymerase, helicase, etc., which are critical for the transcription and replication of the novel coronavirus. Currently, the surface spike glycoprotein of the coronavirus that recognizes the receptor, and the key proteins involved in the replication and transcription processes-3CLpro, PLpro, and RdRp-are four highly attractive targets for antiviral drug development.

Regarding the development of novel coronavirus vaccines, on Dec. 2, the UK first approved emergency use of Pfizer and BioNTech novel coronavirus vaccines. On one hand, the general use effect of the vaccine is not known. On the other hand, the strict low-temperature storage requirement brings great inconvenience to the wide use of the vaccine.

Regarding novel coronavirus drug development, Remdesivir is currently the only approved novel coronavirus drug by the FDA in the United States. Remdesivir is an aminomethyl monophosphate prodrug of an adenosine analog, originally developed by Gilead as an anti-Ebola virus drug. Remdesivir, as an RdRp inhibitor, shows the activity against novel coronavirus at a cell level, but clinical tests show that Remdesivir does not significantly reduce the mortality on a human body. Moreover, some significant side effects have to be noted since the clinically used dose is close to the safe dose.

Therefore, there is still a need for a drug for treating novel coronavirus with good safety, stability, and bioavailability.

### Summary

In order to solve the problems described above, the present invention provides a pharmaceutical composition, a preparation method therefor, and use thereof.

In a first aspect, a pharmaceutical composition is provided.

A pharmaceutical composition, comprising compound SHEN26 or a pharmaceutically acceptable salt thereof, and a pharmaceutically acceptable excipient,

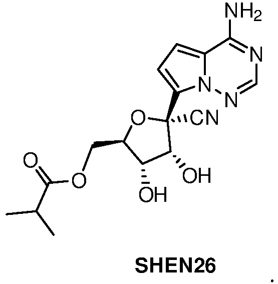

The pharmaceutically acceptable excipient may comprise at least one selected from a diluent, a disintegrant, a binder, a lubricant, and a glidant. In some embodiments, the pharmaceutically acceptable excipient comprises a diluent, a disintegrant, a binder, and a lubricant.

The diluent may comprise at least one selected from microcrystalline cellulose, anhydrous calcium hydrophosphate, and mannitol; preferably, the diluent is microcrystalline cellulose.

The disintegrant may comprise at least one selected from croscarmellose sodium, crospovidone XL-10, sodium carboxymethylcellulose, low-substituted hydroxypropyl cellulose, and pregelatinized starch; preferably, the disintegrant is croscarmellose sodium.

The binder may comprise at least one selected from hydroxypropyl cellulose, povidone K30, hydroxypropyl methylcellulose, and starch; preferably, the binder is hydroxypropyl cellulose.

The lubricant may comprise at least one selected from magnesium stearate, sodium stearyl fumarate, stearic acid, and talc; preferably, the lubricant is magnesium stearate.

The composition may further comprise an external lubricant. The glidant may comprise a glidant selected from colloidal silicon dioxide.

The content of the compound SHEN26 may be 15 wt% to 70 wt%, based on the total mass of the pharmaceutical composition. In some embodiments, the content of the compound SHEN26 is 15 wt%, 20 wt%, 25 wt%, 30 wt%, 35 wt%, 40 wt%, 45 wt%, 50 wt%, 51 wt%, 52 wt%, 53 wt%, 54 wt%, 55 wt%, 56 wt%, 57 wt%, 58 wt%, 59 wt%, 60 wt%, 65 wt%, or 70 wt%, based on the total mass of the pharmaceutical composition. In some embodiments, the content of the compound SHEN26 is 50 wt% to 60 wt%, based on the total mass of the pharmaceutical composition.

The content of the diluent may be 20 wt% to 70 wt%, based on the total mass of the pharmaceutical composition. In some embodiments, the content of the diluent is 20 wt%, 25 wt%, 30 wt%, 31 wt%, 32 wt%, 33 wt%, 34 wt%, 35 wt%, 36 wt%, 37 wt%, 38 wt%, 39 wt%, 40 wt%, 45 wt%, 50 wt%, 55 wt%, 60 wt%, 65 wt%, or 70 wt%, based on the total mass of the pharmaceutical composition. In some embodiments, the content of the diluent is 30 wt% to 40 wt%, based on the total mass of the pharmaceutical composition.

The content of the disintegrant may be 1 wt% to 10 wt%, based on the total mass of the pharmaceutical composition. In some embodiments, the content of the disintegrant is 1 wt%, 2 wt%, 3 wt%, 4 wt%, 5 wt%, 6 wt%, 7 wt%, 8 wt%, 9 wt%, or 10 wt%, based on the total mass of the pharmaceutical composition. In some embodiments, the content of the disintegrant is 2 wt% to 4 wt%, based on the total mass of the pharmaceutical composition.

The content of the binder may be 1 wt% to 10 wt%, based on the total mass of the pharmaceutical composition. In some embodiments, the content of the binder is 1 wt%, 2 wt%, 3 wt%, 4 wt%, 5 wt%, 6 wt%, 7 wt%, 8 wt%, 9 wt%, or 10 wt%, based on the total mass of the pharmaceutical composition. In some embodiments, the content of the binder is 4 wt% to 6 wt%, based on the total mass of the pharmaceutical composition.

The content of the lubricant may be 0.1 wt% to 5 wt%, based on the total mass of the pharmaceutical composition. In some embodiments, the content of the lubricant is 0.1 wt%, 0.1 wt%, 0.2 wt%, 0.3 wt%, 0.4 wt%, 0.5 wt%, 0.6 wt%, 0.7 wt%, 0.8 wt%, 0.9 wt%, or 1 wt%, based on the total mass of the pharmaceutical composition. In some embodiments, the content of the lubricant is 0.5 wt% to 1 wt%, based on the total mass of the pharmaceutical composition.

The content of the external lubricant may be 0.5 wt% to 5 wt%, based on the total mass of the pharmaceutical composition. In some embodiments, the content of the external lubricant is 0.5 wt%, 1.0 wt%, 1.5 wt%, 2.0 wt%, 3.0 wt%, 4.0 wt%, or 5 wt%, based on the total mass of the pharmaceutical composition. In some embodiments, the content of the external lubricant is 0.5 wt% to 1.5 wt%, based on the total mass of the pharmaceutical composition.

In some embodiments, the pharmaceutically acceptable excipient includes a diluent, a disintegrant, a binder, a lubricant, and an external lubricant; the diluent is microcrystalline cellulose, the disintegrant is croscarmellose sodium, the binder is hydroxypropyl cellulose, and the lubricant is magnesium stearate; based on the total mass of the pharmaceutical composition, the content of the compound SHEN26 is 50 wt% to 60 wt%, the content of the diluent is 30 wt% to 40 wt%, the content of the disintegrant is 2 wt% to 4 wt%, the content of the binder is 4 wt% to 6 wt%, the content of the lubricant is 0.5 wt% to 1 wt%, and the content of the external lubricant is 0.5 wt% to 1.5 wt%.

In some embodiments, the pharmaceutically acceptable excipient includes a diluent, a disintegrant, a binder, a lubricant, and an external lubricant; the diluent is microcrystalline cellulose, the disintegrant is croscarmellose sodium, the binder is hydroxypropyl cellulose, and the lubricant is magnesium stearate; based on the total mass of the pharmaceutical composition, the content of the compound SHEN26 is 55.56 wt%, the content of the diluent is 34.94 wt%, the content of the disintegrant is 3 wt%, the content of the binder is 5 wt%, the content of the lubricant is 0.5 wt%, and the content of the external lubricant is 1 wt%.

The dosage form of the composition may be a solid oral dosage form.

The solid oral dosage form may comprise a solid oral dosage form selected from a tablet, a granule, or a capsule.

The specification of the pharmaceutical composition may be 10 mg to 500 mg. In some embodiments, the specification of the pharmaceutical composition is 10 mg, 20 mg, 25 mg, 30 mg, 35 mg, 40 mg, 45 mg, 50 mg, 75 mg, 100 mg, 125 mg, 150 mg, or 200 mg.

In a second aspect, use of the aforementioned pharmaceutical composition is provided.

In some embodiments of the present invention, use of the pharmaceutical composition according to the first aspect in the preparation of a product for preventing, alleviating or treating a coronavirus infection, or replication or propagation of a homologous variant virus thereof, and a cytopathic effect generated therefrom.

The infection may comprise fever, cough, sore throat, pneumonia, acute respiratory infection, severe acute respiratory infection, hypoxic respiratory failure and acute respiratory distress syndrome, sepsis, or septic shock.

In some embodiments of the present invention, use of the pharmaceutical composition according to the first aspect in the preparation of a product for detecting a coronavirus or a homologous variant virus thereof.

The coronavirus may comprise: MHV-A59, HCoV-229E, HCoV-OC43, HCoV-NL63, HCoV-HKU1, SARS-CoV, MERS-CoV, SARS-CoV-2, a mouse hepatitis virus, a feline infectious peritonitis virus, a canine coronavirus, a bovine coronavirus, an avian infectious bronchitis virus, or a porcine coronavirus; preferably, the SARS-CoV-2 comprises a mutant strain or a non-mutant strain of SARS-CoV-2; more preferably, the mutant strain of SARS-CoV-2 comprises SARS-CoV-2 mutant strain B.1, SARS-CoV-2 mutant strain B.1.351, SARS-CoV-2 mutant strain B.1.617.2, SARS-CoV-2 mutant strain C.37, SARS-CoV-2 mutant strain P.1 lineage, SARS-CoV-2 mutant strain B.1.525, SARS-CoV-2 mutant strain B.1.427, or SARS-CoV-2 mutant strain B.1.429.

The pharmaceutical composition may be suitable for use in humans or animals; and/orthe animal comprises bovine, equine, ovine, porcine, canine, feline, rodent, primate, avian, or piscine animal.

In a third aspect, a method for preparing the aforementioned pharmaceutical composition is provided.

In some preferred embodiments of the present invention, a method for preparing the pharmaceutical composition according to the first aspect, comprising: mixing the compound SHEN26 and the pharmaceutically acceptable excipient, performing dry granulation, adding an external lubricant, mixing, and tableting or capsule filling or packaging to give the composition.

In some embodiments of the present invention, a method for preparing the pharmaceutical composition according to the first aspect, comprising: mixing the compound SHEN26 and the pharmaceutically acceptable excipient, performing wet granulation, grinding, drying, dry grinding, adding an external lubricant, mixing, and tableting or capsule filling or packaging to give the composition.

In some preferred embodiments of the present invention, a method for preparing the pharmaceutical composition according to the first aspect, comprising: grinding or pulverizing the compound SHEN26, mixing the compound and the pharmaceutically acceptable excipient, performing dry granulation, adding an external lubricant, mixing, and tableting or capsule filling or packaging to give the composition.

In some embodiments of the present invention, a method for preparing the pharmaceutical composition according to the first aspect, comprising: grinding or pulverizing the compound SHEN26, mixing the compound and the pharmaceutically acceptable excipient, performing wet granulation, grinding, drying, dry grinding, adding an external lubricant, mixing, and tableting or capsule filling or packaging to give the composition.

In the present invention, dry granulation is preferably adopted to prepare the aforementioned pharmaceutical composition. Compared with a direct mixing process, dry granulation is beneficial for avoiding the risk of material delamination and avoiding the problem of uneven mixing.

### Beneficial Effects

Compared with the prior art, one embodiment of the present invention at least has one of the following beneficial technical effects:
(1) The pharmaceutical composition provided by the present invention has the advantages of high dissolution, high release rate, good compatibility of raw and auxiliary materials, good stability, high bioavailability, and the like.
(2) Using the auxiliary material provided by the present invention is beneficial for improving the dissolution, the release rate, the compatibility of raw and auxiliary materials and the stability (the stability includes the stability of property, content, related substance, hygroscopic weight gain, dissolution, release rate and the like) of the obtained pharmaceutical composition and improving the material properties in the preparation process of the pharmaceutical composition.
(3) Using the formula proportion of the SHEN26 compound and/or the formula proportion of each auxiliary material provided by the present invention is beneficial for improving the dissolution, the release rate, the compatibility of raw and auxiliary materials and the stability (the stability includes the stability of property, content, related substance, hygroscopic weight gain, dissolution, release rate and the like) of the obtained pharmaceutical composition and improving the material properties (viscosity, material formability, and particle hardness) in the preparation process of the pharmaceutical composition.

### Definitions of Terms:

In the present invention, "room temperature" presents ambient temperature, may be 10 °C to 40 °C, and may be 20 °C to 30 °C; in some embodiments, the temperature is 22 °C to 28 °C; in some embodiments, the temperature is 24 °C to 26 °C; in some embodiments, the temperature is 25 °C.

The term "specification" refers to the weight of the active ingredient in one unit of formulation (single tablet or single capsule). For example, a 50 mg specification in the present invention refers to 50 mg of the compound SHEN26 in a single tablet or a single capsule.

The term "D90" refers to the particle size corresponding to a 90% cumulative particle size distribution of a sample. Its physical meaning is that particles with particle sizes less than it make up 90%. For example, "D90 is not larger than 100 µm" means "90% of the particles are not larger than 100 µm". D10 refers to the particle size corresponding to a 10% cumulative particle size distribution of a sample; D50 refers to the particle size corresponding to a 50% cumulative particle size distribution of a sample.

The term "open" means that the drug or formulation is placed in an open container without screw capping and sealing, so that the capsule contacts the outside environment.

The term "closed" means that after the drug or formulation is placed in a container, the container is capped and sealed with aluminum foil to isolate the capsule from the outside environment.

In the context of the present invention, all numbers disclosed herein are approximate values, whether or not the word "about" or "approximately" is used. Based on the disclosed numbers, it is possible that the numerical value of each number may have a difference of ±10% or less or a reasonable difference considered by those skilled in the art, such as by ±1%, ±2%, ±3%, ±4%, or ±5%.

"Pharmaceutically acceptable salt", as used herein, refers to organic salts and inorganic salts of the compound of the present invention. Pharmaceutically acceptable salts are well known in the art to which they belong, for example, those described in S. M. Berge et al., describe pharmaceutically acceptable salts in detail in J. Pharmaceutical Sciences, 1977, 66: 1-19. Pharmaceutically acceptable non-toxic salts formed with acids include, but are not limited to, inorganic acid salts formed by reaction with amino groups such as hydrochloride, hydrobromide, phosphate, sulfate, and perchlorate, and organic acid salts such as acetate, oxalate, maleate, tartrate, citrate, succinate, and malonate, or salts obtained by using other methods described in the literature such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, and the like. Salts obtained using appropriate bases include alkali metal, alkaline earth metal, ammonium and N+(C1-4 alkyl)4 salts. The present invention further contemplates quaternary ammonium salts formed from compounds of any N-containing groups. Water-soluble or oil-soluble or dispersable products may be obtained by quaternization. Alkali or alkaline earth metal salts include sodium, lithium, potassium, calcium, magnesium, and the like. Pharmaceutically acceptable salts further include appropriate and non-toxic ammonium, quaternary ammonium salts, and amine cations formed with counterions, such as halide, hydroxide, carboxylate, sulfate, phosphate, nitrate, C1-C8 sulfonate, and aromatic sulfonate.

The term "optional" or "optionally" means that the subsequently described event or case may or may not occur. For example, "optional surfactant" means that the surfactant may or may not be present.

The term "external lubricant" refers to a substance added after granulation to reduce friction between the resulting particles from granulation, to prevent the raw material and the auxiliary material from sticking to the punch surface or to reduce friction between the tablet and the well wall of the punch die.

The term "weight percentage" or "percentage by weight", "wt%", or "w/w%" is defined as follows: The weight of an individual component in a composition is divided by the total weight of all components in the composition and multiplied by 100.

The term "and/or" should be understood to refer to any one of the options or a combination of any two or more of the options.

As used herein, the term "treating" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis.

"Pharmaceutically acceptable" refers to a substance or compound that is, within the scope of sound medical judgment, suitable for contact with the tissues of humans and lower animals without undue toxicity, irritation, allergic response and the like, and is commensurate with a reasonable benefit/risk ratio.

In the specification, terms such as "one embodiment", "some embodiments", "examples", "a specific example", or "some examples", mean that a particular feature, structure, material, or characteristic described in reference to the embodiment or example is included in at least one embodiment or example of the present invention. In this specification, the schematic descriptions of the terms described above do not necessarily refer to the same embodiment or example. Moreover, the specific features, materials, structures, and characteristics described may be combined in any one or more embodiments or examples in an appropriate manner. Moreover, various embodiments or examples and features of various embodiments or examples described in this specification can be combined by one skilled in the art to the extent that they do not contradict each other.

In the present application, a "composition" may be conveniently presented in unit dose form and may be prepared by any of the methods well known in the pharmaceutical art. All the methods include the step of combining the active ingredient with the carrier which constitutes one or more accessory ingredients. Generally, compositions are prepared by uniformly and sufficiently combining the active compound with a liquid carrier, a finely divided solid carrier, or both.

In the present invention, "compounds SHEN26", "SHEN26" and "SHEN26 compound" all have the same meaning.

### Detailed Description

In order to make the technical solutions of the present invention better understood by those skilled in the art, some non-limiting examples are further disclosed below to further explain the present invention in detail.

The reagents used in the present invention are either commercially available or can be prepared by the methods described herein.

"#0" indicates capsule shell #0. "#4" indicates capsule shell #4. "API" refers to a drug substance and in the following specific examples refers to the compound SHEN26. "rpm" refers to the unit of rotational speed "revolutions per minute".

### Dissolution measurement method

Unless otherwise indicated, the dissolution measurement method used in the formulation studies of this product is shown in Table 1.

**Table 1. Dissolution method**

| | |
|---|---|
| **Dissolution device** | Chinese Pharmacopoeia, 2020 Edition, 0931, basket method |
| **Dissolution medium** | pH 2.0 HCl |
| **Volume of dissolution medium** | 900 mL |
| **Temperature of dissolution medium** | 37 ± 0.5 °C |
| **Rotation speed of basket** | 75 rpm |
| **Sampling time point** | 5 min, 15 min, 30 min, 45 min, 60 min, and 75 min (60-75 min was the ultimate rotation speed test, and the rotation speed was 250 rpm) |

### Example 1: Investigation of Preparation Process

### (1) Direct mixing process

Formula: See Table 2.

Operation: directly mixing a SHEN26 compound with microcrystalline cellulose 102, mannitol 100SD, croscarmellose sodium VIVASOL^{®} and magnesium stearate LIGAMED MF-2-V to give a mixed powder, and filling capsules to give a SHEN26 compound capsule.

Detection of micromeritic properties: The micromeritic properties of the SHEN26 compound raw material and the micromeritic properties of the mixed powder obtained by directly mixing the SHEN26 compound and other auxiliary materials were detected. The results are shown in Table 3.

**Table 2: Formula composition of direct mixing process**

| Process | Direct mixing | | |
|---|---|---|---|
| API particle size (µm) | D10: 60.5, D50: 141, D90: 401 | | |
| Batch No. | 37167-019A | 37167-019B | N/A |
| Specification | 50 mg | 200 mg | N/A |
| Formula component | mg/capsule | mg/capsule | wt% |
| Content | | | |
| SHEN26 | 50.0 | 200.0 | 55.56 |
| Microcrystalline cellulose 102 | 24.0 | 96.0 | 26.67 |
| Mannitol 100SD | 12.4 | 49.6 | 13.78 |
| Croscarmellose sodium VIVASOL^{®} | 2.7 | 10.8 | 3.00 |
| Magnesium stearate LIGAMED MF-2-V | 0.9 | 3.6 | 1.00 |
| Total | 90.0 | 360.0 | 100.00 |

| Capsule shell | | | |
|---|---|---|---|
| Gelatin empty capsule 4# opaque white | 38.0 | N/A | 1 capsule |
| Gelatin empty capsule 0# opaque white | N/A | 96.0 | 1 capsule |

**Table 3: Results of micromeritic properties of API and formula**

| **Batch No.** | **Bulk density (g/mL)** | **Tap density (g/mL)** | **Carr index (%)** | **Angle of repose (°)** |
|---|---|---|---|---|
| SHEN26 compound raw material (batch No. A16305-006P2) | 0.53 | 0.73 | 27.40 | 40.98 |
| 37167-019A/B | 0.53 | 0.66 | 19.70 | 36.32 |

Analysis of results: It can be seen from the above data that the drug substance had relatively good fluidity. After the drug substance was directly mixed with the auxiliary material, the Carr index was further reduced. Moreover, the API particle size was relatively large, and the risk of delamination existed in the production process. Therefore, the dry granulation process was further investigated.

### (2) Dry granulation process

Formula: See Table 4.

Operation: mixing the compound SHEN26 with an internal auxiliary material, performing dry granulation, adding an external auxiliary material, mixing, and filling capsules to give a capsule of the compound SHEN26.

**Table 4: Formula composition**

| **Process** | | | | **Direct mixing** | | **Dry granulation** | |
|---|---|---|---|---|---|---|---|
| **Batch No.** | | | | **37167-019A/B** | | **37167-036A/B** | |
| **Batch** | | | | **167 capsules (based on 200 mg specification)** | | **100 capsules (based on 200 mg specification)** | |
| **API particle size (µm)** | | | | **D10: 60.5, D50: 141, D90: 401** | | | |
| **Description** | | | | **13.78% mannitol 100SD and 1.0% magnesium stearate** | | **5% hydroxypropyl cellulose EXF, 0.5% magnesium** | |
| | | **(internal), no hydroxypropyl cellulose EXF and magnesium stearate (external)** | | | **stearate (internal) 1.0% magnesium stearate (external), removing mannitol 100SD** | | |
| **Specification** | | **50 mg** | **200 mg** | **N/A** | **50 mg** | **200 mg** | **N/A** |
| **Formula component** | | **mg/capsule** | **mg/capsule** | **wt%** | **mg/capsule** | **mg/capsule** | **wt%** |
| Content | | | | | | | |
| Drug substance | SHEN26 | 50.0 | 200.0 | 55.56 | 50.00 | 200.00 | 55.56 |
| Internal auxiliary material | Microcrystalline cellulose 102 | 24.0 | 96.0 | 26.67 | 31.45 | 125.80 | 34.94 |
| | Mannitol 100SD | 12.4 | 49.6 | 13.78 | N/A | N/A | N/A |
| | Croscarmellose sodium VIVASOL^{®} | 2.7 | 10.8 | 3.00 | 2.70 | 10.80 | 3.00 |
| | Hydroxypropyl cellulose EXF | N/A | N/A | N/A | 4.50 | 18.00 | 5.00 |
| | Magnesium stearate LIGAMED MF-2-V (internal) | 0.9 | 3.6 | 1.00 | 0.45 | 1.80 | 0.50 |
| External auxiliary material | Magnesium stearate LIGAMED MF-2-V (external) | N/A | N/A | N/A | 0.90 | 3.60 | 1.00 |
| Total | | 90.00 | 360.00 | 100.00 | 90.00 | 360.00 | 100.00 |

| Capsule shell | | | | | | | |
|---|---|---|---|---|---|---|---|
| Gelatin empty capsule 4# opaque white | | 38.0 | N/A | 1 capsule | 38.0 | N/A | 1 capsule |
| Gelatin empty capsule 0# opaque white | | N/A | 96.0 | 1 capsule | N/A | 96.0 | 1 capsule |

**Dissolution detection:** The direct mixing process formula was compared with the dry granulation process formula, and the dissolution results are shown in Table 5.

**Table 5: Dissolution results**

| **Process** | **Batch No./specification** | **Dissolution** | **Dissolution results (%) (n = 3)** | | | | | |
|---|---|---|---|---|---|---|---|---|
| | | | **Dissolution method: basket method** | | | | | |
| | | | **dissolution medium: pH 2.0 HCl** | | | | | |
| | | | **Volume of medium: 900 mL rotation speed: 75 rpm** | | | | | |
| | | | **5 min** | **15 min** | **30 min** | **45 min** | **60 min** | **75 min** |
| Direct mixing | 37167-019B (200mg) | **Dissolution** | 32 | 95 | 105 | 106 | 106 | 106 |
| | | RSD% | 37.3 | 12.9 | 3.5 | 2.9 | 2.7 | 2.5 |
| Dry granulation | 37167-036B (200mg) | **Dissolution** | 55 | 93 | 95 | 95 | 96 | 96 |
| | | RSD% | 5.6 | 5.1 | 3.7 | 3.0 | 2.5 | 2.0 |

Analysis of results: It can be seen from the above data that the dissolution of the capsules of the two processes met the requirements, but in the directly mixing, after capsule filling and 30 min after dissolution, the content reached a relatively high value, which indicates that the direct mixing process may have a risk of delamination, so that the dry granulation process was finally determined for formula development.

### Example 2: Investigation of Binder Amount

Formula: See Table 6A, Table 6B, and Table 6C.

**Table 6A: Formula composition**

| Batch No. | | 37167-032A-1 | 37167-032B-1 | NA |
|---|---|---|---|---|
| Description | | No hydroxypropyl cellulose | | |
| Specification | | 50 mg | 200mg | N/A |
| Formula component | | mg/capsule | mg/capsule | wt% |
| Drug substance | SHEN26 | 50 | 200 | 55.56 |
| Internal auxiliary material | Microcrystalline cellulose 102 | 23.75 | 95 | 26.39 |
| | Mannitol 100SD | 12.2 | 48.8 | 13.56 |
| | Croscarmellose sodium VIVASOL^{®} | 2.7 | 10.8 | 3 |
| | Hydroxypropyl cellulose EXF | N/A | N/A | N/A |
| | Magnesium stearate (internal) | 0.45 | 1.8 | 0.5 |
| External auxiliary material | Magnesium stearate (external) | 0.9 | 3.6 | 1 |
| Total | | 90 | 360 | 100 |

**Table 6B: Formula composition**

| Batch No. | | 37167-032A-2 | 37167-032B-2 | NA |
|---|---|---|---|---|
| Description | | 3% hydroxypropyl cellulose | | |
| Specification | | 50 mg | 200mg | N/A |
| Formula component | | mg/capsule | mg/capsule | wt% |
| Drug substance | SHEN26 | 50 | 200 | 53.94 |
| Internal auxiliary material | Microcrystalline cellulose 102 | 23.75 | 95 | 25.62 |
| | Mannitol 100SD | 12.2 | 48.8 | 13.16 |
| | Croscarmellose sodium VIVASOL^{®} | 2.7 | 10.8 | 2.91 |
| | Hydroxypropyl cellulose EXF | 2.7 | 10.8 | 2.91 |
| | Magnesium stearate (internal) | 0.45 | 1.8 | 0.49 |
| External auxiliary material | Magnesium stearate (external) | 0.9 | 3.6 | 0.97 |
| Total | | 92.7 | 370.8 | 100 |

**Table 6C: Formula composition**

| Batch No. | | 37167-032A | 37167-032B | NA |
|---|---|---|---|---|
| Description | | 5% hydroxypropyl cellulose | | |
| Specification | | 50 mg | 200mg | N/A |
| Formula component | | mg/capsule | mg/capsule | wt% |
| Drug substance | SHEN26 | 50 | 200 | 55.56 |
| Internal auxiliary material | Microcrystalline cellulose 102 | 20.95 | 83.8 | 23.28 |
| | Mannitol 100SD | 10.5 | 42 | 11.67 |
| | Croscarmellose sodium VIVASOL^{®} | 2.7 | 10.8 | 3 |
| | Hydroxypropyl cellulose EXF | 4.5 | 18 | 5 |
| | Magnesium stearate (internal) | 0.45 | 1.8 | 0.5 |
| External auxiliary material | Magnesium stearate (external) | 0.9 | 3.6 | 1 |
| Total | | 90 | 360 | 100 |

Preparation method: pulverizing the compound SHEN26, mixing the compound with an internal auxiliary material, performing dry granulation, adding an external auxiliary material, mixing, and filling capsules to give a capsule of the compound SHEN26.

Observation of phenomenon: During the dry granulation, the material viscosity, the formability and the particle hardness were observed, and the results are shown in Table 7.

**Table 7: Phenomenon of dry granulation**

| **Formula batch No.** | **Process parameter** | | | **Phenomenon** |
|---|---|---|---|---|
| | **Roll pressure (kg/cm²)** | **Feed rate (rpm)** | **Roll speed (rpm)** | |
| 37167-032A-1 and 37167-032B-1 | 20-30 | 0 | 0.96 | No adhesion with the roller, relatively poor formability of the band, and relatively soft particles |
| | 45-55 | 0 | 0.96 | No adhesion with the roller, relatively poor formability of the band, and relatively soft particles |
| 37167-032A-2 and 37167-032B-2 | 20-30 | 0 | 0.96 | No adhesion with the roller, relatively poor formability of the band, and relatively soft particles |
| | 45-55 | 0 | 0.96 | No adhesion with the roller, relatively poor formability of the band, and relatively soft particles |
| 37167-032A and 37167-032B | 20-30 | 0 | 0.96 | Slight adhesion with the roller, relatively good formability of the band, and moderate particle hardness |

Analysis of results: It can be seen from the above dry granulation phenomena, during the dry granulation of the formulas 37167-032A-1 and 37167-032B-1, the bands had a relatively poor formability; when the amount of the binder hydroxypropyl cellulose EXF was 3%, the bands of the formulas 37167-032A-2 and 37167-032B-2 had a poor formability, and the particles were relatively soft; after the amount of the binder hydroxypropyl cellulose EXF was adjusted to 5%, although the formulas 37167-032A and 37167-032B had a slight roller adhesion phenomenon, the bands had a relatively good formability, and the particle hardness was moderate, so that 5% hydroxypropyl cellulose EXF was selected as the binder.

### Example 3: Investigation of Filler Type

Formula: See Table 8.

**Table 8: Formula composition**

| **Description** | | **Microcrystalline cellulose 102:mannitol 100SD = 2:1** | | | Only microcrystalline cellulose **102 Removing mannitol 100SD** | | |
|---|---|---|---|---|---|---|---|
| **Batch No.** | | **37167-023A** | **37167-023B** | **N/A** | **37167-036A** | **37167-036B** | **N/A** |
| **Specification** | | **50 mg** | **200mg** | **N/A** | **50 mg** | **200mg** | **N/A** |
| **Formula component** | | **mg/capsule** | **mg/capsule** | **wt%** | **mg/capsule** | **mg/capsule** | **wt%** |
| **Content** | | | | | | | |
| Drug substance | SHEN26 | 50.00 | 200.0 | 55.56 | 50.00 | 200.00 | 55.56 |
| Internal auxiliary material | Microcrystalline cellulose 102 | 20.95 | 83.80 | 23.28 | 31.45 | 125.80 | 34.94 |
| | Mannitol 100SD | 10.50 | 42.00 | 11.67 | N/A | N/A | N/A |
| | Croscarmellose sodium VIVASOL^{®} | 2.70 | 10.80 | 3.00 | 2.70 | 10.80 | 3.00 |
| | Hydroxypropyl cellulose EXF | 4.50 | 18.00 | 5.00 | 4.50 | 18.00 | 5.00 |
| | Magnesium stearate LIGAMED MF-2-V (internal) | 0.45 | 1.80 | 0.50 | 0.45 | 1.80 | 0.50 |
| External auxiliary material | Magnesium stearate LIGAMED MF-2-V (external) | 0.90 | 3.60 | 1.00 | 0.90 | 3.60 | 1.00 |
| Total | | 90.00 | 360.0 | 100.00 | 90.00 | 360.00 | 100.00 |

| Capsule shell | | | | | | | |
|---|---|---|---|---|---|---|---|
| Gelatin empty capsule 4# opaque white | | 38.0 | N/A | 1 capsule | 38.0 | N/A | 1 capsule |
| Gelatin empty capsule 0# opaque white | | N/A | 96.0 | 1 capsule | N/A | 96.0 | 1 capsule |

Preparation method: pulverizing the compound SHEN26, mixing the compound with a pharmaceutically acceptable excipient, performing dry granulation, adding an external lubricant, mixing, and filling capsules to give a capsule of the compound SHEN26.

Observation of phenomenon: During the dry granulation, the material viscosity, the formability and the particle hardness were observed, and the results are shown in Table 9.

Dissolution detection: The compound SHEN26 capsules obtained in Example 3 were separately detected for the dissolution, and the results are shown in Table 10.

**Table 9: Phenomenon of dry granulation**

| **Formula** | **Process parameter** | | | **Phenomenon** |
|---|---|---|---|---|
| | **Roll pressure (kg/cm²)** | **Feed rate (rpm)** | **Roll speed (rpm)** | |
| 37167-023A and 37167-023B | 20-30 | 0 | 0.96 | Slight adhesion with the roller, relatively good formability of the band, and moderate particle hardness |
| 37167-036A and 37167-036B | 20-30 | 0 | 0.96 | No adhesion with the roller, relatively good formability of the band, and moderate particle hardness |

**Table 10: Dissolution results**

| **Batch No./specification** | **Dissolution** | **Dissolution results (%) (n = 3)** | | | | | |
|---|---|---|---|---|---|---|---|
| | | **Dissolution method: basket method dissolution medium: pH 2.0 HCl** | | | | | |
| | | **Volume of medium: 900 mL rotation speed: 75 rpm** | | | | | |
| | | **5 min** | **15 min** | **30 min** | **45 min** | **60 min** | **75 min** |
| 37167-036B (200 mg specification) | Dissolution | 55 | 93 | 95 | 95 | 96 | 96 |
| | RSD% | 5.6 | 5.1 | 3.7 | 3.0 | 2.5 | 2.0 |

Analysis of results: It can be seen from the above dry granulation phenomena, when the ratio of microcrystalline cellulose 102 to mannitol 100SD was 2:1, the formulas 37167-023A and 37167-023B had a slight roller adhesion phenomenon during the dry granulation, the bands had a relatively good formability, and the particle hardness was moderate; after the filler mannitol was removed, the 37167-036A/B formula did not show a roller adhesion phenomenon during the dry granulation, the bands had a relatively good formability, the particle hardness was moderate, and the dissolution met the requirements, so that microcrystalline cellulose 102 was determined to be selected as the filler.

### Example 4: Investigation of Particle Size of Drug Substance

Formula: See Table 11.

**Table 11: Formula composition**

| **Drug substance batch No.** | | **A16305-036P1** | | | | | |
|---|---|---|---|---|---|---|---|
| **Pretreatment of drug substance** | | **Sieving with 40-mesh sieve (280 µm)** | | | **Sieving with 60-mesh sieve (450 µm)** | | |
| **Particle size of drug substance after pretreatment** | | **D10:5.982µm** | | | **D10:3.317µm** | | |
| | | **D50:75.714µm** | | | **D50:35.271µm** | | |
| | | **D90:327.667µm** | | | **D90:195.440µm** | | |
| **Finished product batch No.** | | **37167-045A-1** | **37167-045B-1** | **NA** | **37167-045A-2** | **37167-045B-2** | **NA** |
| **Specification** | | **50 mg** | **200mg** | **Percentage** | **50 mg** | **200mg** | **Percentage** |
| **Formula component** | | **mg/capsule** | **mg/capsule** | **wt%** | **mg/capsule** | **mg/capsule** | **wt%** |
| Content | | | | | | | |
| Drug substance | SHEN26 | 50 | 200 | 55.56 | 50 | 200 | 55.56 |
| Internal auxiliary material | Microcrystalline cellulose 102 | 31.45 | 125.8 | 34.94 | 31.45 | 125.8 | 34.94 |
| | Croscarmellose sodium VIVASOL^{®} | 2.7 | 10.8 | 3 | 2.7 | 10.8 | 3 |
| | Hydroxypropyl cellulose EXF | 4.5 | 18 | 5 | 4.5 | 18 | 5 |
| | Magnesium stearate LIGAMED MF-2-V (internal) | 0.45 | 1.8 | 0.5 | 0.45 | 1.8 | 0.5 |
| External auxiliary material | Magnesium stearate LIGAMED MF-2-V (external) | 0.9 | 3.6 | 1 | 0.9 | 3.6 | 1 |
| Total | | 90 | 360 | 100 | 90 | 360 | 100 |

| Capsule shell | | | | | | | |
|---|---|---|---|---|---|---|---|
| Gelatin empty capsule 4# opaque white | | 38 | N/A | 1 capsule | 38 | N/A | 1 capsule |
| Gelatin empty capsule 0# opaque white | | N/A | 96 | 1 capsule | N/A | 96 | 1 capsule |

Preparation method: pulverizing the compound SHEN26, sieving the compound (sieving separately with a 40-mesh sieve and a 60-mesh sieve), mixing with a pharmaceutically acceptable excipient, performing dry granulation, adding an external lubricant, mixing, and filling capsules to give a capsule of the compound SHEN26.

Observation of phenomenon: After dry granulation, the micromeritic properties were examined, and the results are shown in Table 12.

Dissolution detection: The compound SHEN26 capsules obtained in Example 4 were separately detected for the dissolution, and the results are shown in Table 13.

**Table 12: Results of micromeritic properties of dry granulation formula**

| **Batch No.** | **Bulk density (g/mL)** | **Tap density (g/mL)** | **Carr index (%)** | **Angle of repose (°)** |
|---|---|---|---|---|
| 37167-045A/B-2 | 0.5008 | 0.7033 | 28.79 | 39.88 |

**Table 13: Dissolution results**

| **Drug substance Particle size (µm)** | **Batch No./specification** | **Dissolution** | **Dissolution results (%) (n = 3)** | | | | | |
|---|---|---|---|---|---|---|---|---|
| | | | **Dissolution method: basket method dissolution medium: pH 2.0 HCl** | | | | | |
| | | | **Volume of medium: 900 mL rotation speed: 75 rpm** | | | | | |
| | | | **5 min** | **15 min** | **30 min** | **45 min** | **60 min** | **75 min** |
| D10: 5.982 | 37167-045A-1 50 mg | Dissolution | 48 | 94 | 102 | 103 | 103 | 102 |
| D50: 75.714 | | RSD% | 6.0 | 2.0 | 0.5 | 0.5 | 0.5 | 0.5 |
| D90: 327.667 | 37167-045B-1 200 mg | Dissolution | 33 | 75 | 96 | 101 | 102 | 102 |
| | | RSD% | 18.7 | 7.4 | 1.2 | 1.4 | 1.8 | 1.5 |
| D10: 3.317 | 37167-045A-2 50 mg | Dissolution | 70 | 98 | 101 | 101 | 101 | 101 |
| D50: 35.271 | | RSD% | 3.9 | 3.4 | 2.1 | 2.2 | 2.0 | 2.0 |
| D90: 195.440 | 37167-045B-2 200 mg | Dissolution | 49 | 86 | 96 | 98 | 98 | 97 |
| | | RSD% | 5.8 | 3.7 | 2.0 | 1.3 | 1.4 | 1.3 |

It can be seen from the above results that the final formula of dry granulation had relatively good fluidity, and the particle size D90 of the drug substance was in the range of 195.440 µm to 327.667 µm, which did not affect the dissolution behavior of the product. Considering the influence of large particle size of the drug substance on the process, the drug substance was pulverized and sieved with a 60-mesh sieve, and then the process was investigated.

### Example 5: Preparation of Compound SHEN26 Capsule

Formula: See Table 14.

**Table 14: Unit dose product composition of SHEN26 capsule**

| Formula component | | Batch No. 37167-054A | Batch No. 37167-054B | Ratio % | Effect |
|---|---|---|---|---|---|
| | | 50 mg Specification | 200 mg Specification | | |
| | | mg/capsule | | | |
| Drug substance | Compound SHEN26 | 50.00 | 200.00 | 55.56 | Activity Component |
| Internal auxiliary material | Microcrystalline cellulose 102 | 31.45 | 125.80 | 34.94 | Diluent |
| | Croscarmellose sodium VIVASOL^{®} | 2.70 | 10.80 | 3.00 | Disintegrant |
| | Hydroxypropyl cellulose EXF | 4.50 | 18.00 | 5.00 | Binder |
| | Magnesium stearate LIGAMED MF-2-V (internal) | 0.45 | 1.80 | 0.50 | Lubricant |
| External auxiliary material | Magnesium stearate LIGAMED MF-2-V (external) | 0.90 | 3.60 | 1.00 | Lubricant |
| Total | | 90.00 | 360.00 | 100.00 | N/A |
| Gelatin empty capsule 4# opaque white | | 38.0 | N/A | 1 capsule | Capsule shell |
| Gelatin empty capsule 0# opaque white | | N/A | 96.0 | 1 capsule | Capsule shell |

| | | | | | |
|---|---|---|---|---|---|
| Note: *The ingredients of the gelatin empty capsules, 4# opaque white (cap color No. 44.801, body color No. 44.801) and 0# opaque white (cap color No. 44.801, body color No. 44.801)-CN, US are titanium white and gelatin. N/A: not applicable. | | | | | |

Preparation method: grinding or pulverizing the compound SHEN26, mixing the compound with a pharmaceutically acceptable excipient, performing dry granulation, adding an external lubricant, mixing, and filling capsules to give a capsule of the compound SHEN26.

Dissolution detection: The compound SHEN26 capsules obtained in Example 5 were separately detected for the dissolution, and the results are shown in Table 15.

**Table 15: Dissolution results**

| **Batch No./specification** | **Dissolution** | **Dissolution results (%) (n = 3)** | | | | | |
|---|---|---|---|---|---|---|---|
| | | **Dissolution method: basket method dissolution medium: pH 2.0 HCl** | | | | | |
| | | **Volume of medium: 900 mL rotation speed: 75 rpm** | | | | | |
| | | **5 min** | **15 min** | **30 min** | **45 min** | **60 min** | **75 min** |
| 37167-054A 50 mg | **Dissolution** | 63 | 101 | 101 | 102 | 102 | 101 |
| | RSD% | 31.7 | 1.0 | 1.3 | 1.3 | 1.1 | 1.1 |
| 37167-054B | **Dissolution** | 55 | 97 | 98 | 99 | 99 | 99 |
| 200 mg | RSD% | 14.7 | 2.5 | 2.9 | 3.0 | 3.2 | 3.2 |

Analysis of results: The compound SHEN26 capsules obtained by the formula and the preparation process provided by the present invention had quick and complete dissolution and release.

### Example 6: Compatibility of Raw Material and Auxiliary Material

Operation: Common auxiliary materials of solid oral dosage forms were selected to be mixed with the SHEN26 drug substance in different ratios, and the mixtures were placed under different conditions and separately taken out at different time points. The compatibility of the drug substance and the auxiliary materials was judged by taking the appearance, the hygroscopic weight gain, the content and the related substance as indexes. For the ratio of the raw and auxiliary materials, according to "Basic Technical Guidelines for Research of Chemical Drug Formulation" and the specification of the formulation, the auxiliary material with a relatively large amount was determined according to a ratio of main drug:diluent = 1:5, the auxiliary material with a moderate amount (such as croscarmellose sodium) was mixed according to a ratio of main drug:disintegrant = 1:1, and the auxiliary material with a relatively small amount (such as magnesium stearate) was mixed according to a ratio of main drug:lubricant = 5:1. The manufacturer information of the raw and auxiliary materials in the compatibility experiment and the ratio of raw and auxiliary materials are shown in Table 16.

Placement conditions: The samples were placed under the experimental conditions of high temperature (60 °C), high humidity (25 °C/90% ± 5% RH), acceleration (40 °C/75% ± 5% RH), and illumination (visible light 4500 Lux ± 500 Lux, near ultraviolet light 85 µw/cm²), and the samples were taken for investigation on day 0, day 10, and day 30. The appearance, the hygroscopic weight gain, the content and the related substance were detected.

**Table 16: Investigation of compatibility of raw material and auxiliary material**

| **Material** | | **Manufacturer** | **Effect** | **Ratio of raw material and auxiliary material (API:auxiliary material)** |
|---|---|---|---|---|
| API | SHEN26 | Pharmaron (Ningbo) Co., Ltd. | Active ingredient | N/A |
| Auxiliary material | Microcrystalline cellulose 102 | Microcellulose Weissenborn GmbH + CO. KG | Diluent | 1:5 |
| | Croscarmellose sodium | CHP CarbohydraPirna GmbH & CO. KG | Disintegrant | 1:1 |
| | Hydroxypropyl cellulose EXF | Ashland Specialty Ingredients G.P | Binder | 1:1 |
| | Magnesium stearate LIGAMED MF-2-V | Peter Greven Nederland C.V. | Lubricant | 5:1 |
| | Gelatin empty capsule 3# rich yellow | Suzhou Capsugel Co., Ltd. | Capsule shell | 1:1 |

Note: Since the opaque rich yellow components of the gelatin empty capsule 3# were the same as the opaque white empty capsule components of the gelatin empty capsules 4# and 0# except for the pigment, which were gelatin and titanium white, only the opaque rich yellow capsule shell of the gelatin empty capsule 3# was investigated in the experiment.

Results: The auxiliary materials described above had good compatibility with SHEN26, and had no significant changes in the appearance, hygroscopic weight gain, content and related substance under each placement condition for 30 days.

### Example 7: Stability Investigation

Operation: The compound SHEN26 capsules obtained in Example 5 having batch Nos. 37167-054A and 37167-054B were packed as described in Table 17 and Table 18, then separately placed under illumination (open), 40 °C/75% RH (open), and 40 °C/75% RH (closed) conditions for 0 day, 10 days, and 30 days, and detected for the dissolution and related substance. The results are shown in Table 19 and Table 20.

**Table 17: Packaging information (bottle package)**

| **Sample name** | **SHEN26 capsule, 50 mg** | **SHEN26 capsule, 200 mg** |
|---|---|---|
| Batch No. | 37167-054A | 37167-054B |
| Package specification | 20 capsules/bottle | 20 capsules/bottle |
| Sample package | Medical high-density polyethylene bottle for oral solid 45 mL | Medical high-density polyethylene bottle for oral solid 75 mL |
| | Medical child safety cap for oral solid CRC33-4 | Medical child safety cap for oral solid CRC33-4 |
| | Medical high-density polyethylene desiccant canister for oral solid, 1 g/canister | Medical high-density polyethylene desiccant canister for oral solid, 1 g/canister |

**Table 18: Packaging information (double aluminum package)**

| **Sample name** | **SHEN26 capsule, 50 mg** | **SHEN26 capsule, 200 mg** |
|---|---|---|
| Batch No. | 37167-054A | 37167-054B |
| Package specification | 4 capsules/strip | 4 capsules/strip |
| Sample package | Solid medical composite hard sheet formed from polyamide/aluminum/polyvinyl chloride by cold stamping | Solid medical composite hard sheet formed from polyamide/aluminum/polyvinyl chloride by cold stamping |
| | Medical aluminum foil | Medical aluminum foil |

**Table 19: Stability results-dissolution**

| **Batch No./specification** | **Package** | **Placement condition and time point** | **Dissolution** | **Dissolution results (%) (n = 3)** | | | | | |
|---|---|---|---|---|---|---|---|---|---|
| | | | | **Dissolution method: basket method** | | | | | |
| | | | | **dissolution medium: pH 2.0 HCl** | | | | | |
| | | | | **Volume of medium: 900 mL rotation** | | | | | |
| | | | | **speed: 75 rpm** | | | | | |
| | | | | **5 min** | **15 min** | **30 min** | **45 min** | **60 min** | **75 min** |
| 37167-054A 50 mg | N/A | 0 day | Dissolution | 63 | 101 | 101 | 102 | 102 | 101 |
| | | | RSD% | 31.7 | 1.0 | 1.3 | 1.3 | 1.1 | 1.1 |
| | Bottle package | 40 °C/75% RH - open (10 days) | Dissolution | 43 | 100 | 103 | 103 | 103 | 103 |
| | | | RSD% | 15.8 | 3.2 | 2.7 | 2.6 | 2.8 | 2.6 |
| | | 40 °C/75% RH - open (30 days) | Dissolution | 41 | 97 | 99 | 100 | 100 | 100 |
| | | | RSD% | 24.5 | 3.9 | 1.8 | 2.1 | 1.7 | 1.7 |
| | Double aluminum | 40 °C/75% RH - (10 days) | Dissolution | 68 | 97 | 98 | 98 | 99 | 99 |
| | | | RSD% | 17.2 | 3.7 | 2.5 | 2.3 | 2.4 | 2.5 |
| | | 40 °C/75% RH - (30 days) | Dissolution | 66 | 103 | 103 | 103 | 103 | 103 |
| | | | RSD% | 9.1 | 3.1 | 3.4 | 3.1 | 3.5 | 3.3 |
| 37167-054B 200 mg | N/A | 0 day | Dissolution | 55 | 97 | 98 | 99 | 99 | 99 |
| | | | RSD% | 14.7 | 2.5 | 2.9 | 3.0 | 3.2 | 3.2 |
| | Bottle package | 40 °C/75% RH - open (10 days) | Dissolution | 37 | 96 | 99 | 99 | 99 | 99 |
| | | | RSD% | 3.7 | 0.5 | 0.8 | 0.4 | 0.5 | 0.3 |
| | | 40 °C/75% RH - open (30 days) | Dissolution | 34 | 92 | 98 | 98 | 99 | 99 |
| | | | RSD% | 44.7 | 9.2 | 2.7 | 2.5 | 2.4 | 2.4 |
| | Double aluminum | 40 °C/75% RH (10 days) | Dissolution | 58 | 94 | 97 | 97 | 98 | 98 |
| | | | RSD% | 12.2 | 0.5 | 0.4 | 0.3 | 0.1 | 1.0 |
| | | 40 °C/75% RH - (30 days) | Dissolution | 59 | 100 | 102 | 102 | 102 | 102 |
| | | | RSD% | 14.6 | 3.1 | 1.4 | 1.5 | 1.5 | 1.5 |

**Table 20: Stability results-related substance**

| **Batch NoJspecification** | **Package** | **Condition** | **Related substance %** | | | | |
|---|---|---|---|---|---|---|---|
| | | | **RRT (relative retention time)** | | | | **Total impurity %** |
| | | | **0.37** | **0.54** | **1.03** | **1.30** | |
| API A16305-036P1 | N/A | N/A | 0.26 | 0.35 | 0.15 | 0.05 | 0.81 |
| 37167-054A (50 mg) | N/A | 0 day | 0.25 | 0.35 | 0.16 | 0.05 | 0.81 |
| | N/A | Illumination (10 days) | 0.26 | 0.35 | 0.16 | 0.05 | 0.82 |
| | Bottle package | 40 °C/75% RH - open (10 days) | 0.26 | 0.34 | 0.16 | 0.05 | 0.81 |
| | | 40 °C/75% RH - open (30 days) | 0.26 | 0.33 | 0.16 | 0.05 | 0.80 |
| | | 40 °C/75% RH - closed (10 days) | 0.25 | 0.35 | 0.16 | 0.06 | 0.82 |
| | | 40 °C/75% RH - closed (30 days) | 0.25 | 0.34 | 0.16 | 0.05 | 0.80 |
| | Double aluminum | 40 °C/75% RH (10 days) | 0.25 | 0.35 | 0.16 | 0.05 | 0.81 |
| | | 40 °C/75% RH (30 days) | 0.25 | 0.34 | 0.16 | 0.05 | 0.80 |
| 37167-054B (200 mg) | N/A | 0 day | 0.26 | 0.35 | 0.16 | 0.06 | 0.83 |
| | N/A | Illumination (10 days) | 0.28 | 0.35 | 0.16 | 0.06 | 0.85 |
| | Bottle package | 40 °C/75% RH - open (10 days) | 0.28 | 0.33 | 0.16 | 0.05 | 0.82 |
| | | 40 °C/75% RH - open (30 days) | 0.27 | 0.33 | 0.16 | 0.05 | 0.81 |
| | | 40 °C/75% RH - closed (10 days) | 0.27 | 0.35 | 0.16 | 0.05 | 0.83 |
| | | 40 °C/75% RH - closed (30 days) | 0.25 | 0.34 | 0.16 | 0.05 | 0.80 |
| | Double aluminum | 40 °C/75% RH (10 days) | 0.25 | 0.35 | 0.16 | 0.05 | 0.81 |
| | | 40 °C/75% RH (30 days) | 0.25 | 0.35 | 0.16 | 0.05 | 0.81 |

### Analysis of results:

It can be seen from the above dissolution data that after the 50 mg specification and 200 mg specification capsules packaged in bottles or double aluminum were placed for 30 days under the conditions of illumination (open), 40 °C/75% RH (open) and 40 °C/75% RH (closed), the dissolution and related substance did not change significantly, and the compound SHEN26 capsules provided by the present invention had good stability.

The method of the present invention has been described by preferred embodiments. It will be apparent to those skilled in the art that the method and application described herein can be implemented and applied with modification or with appropriate modification and combination within the content, spirit, and scope of the present invention. Those skilled in the art can modify the process parameters appropriately in view of the disclosure herein. It is specifically noted that all such substitutions and modifications will be apparent to those skilled in the art and are intended to be included herein.

## Claims

1. A pharmaceutical composition, comprising compound SHEN26 or a pharmaceutically acceptable salt thereof, and a pharmaceutically acceptable excipient,

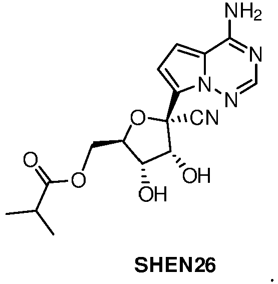

2. The pharmaceutical composition according to claim 1, wherein the pharmaceutically acceptable excipient comprises at least one selected from a diluent, a disintegrant, a binder, a lubricant, and a glidant; optionally, the pharmaceutically acceptable excipient comprises a diluent, a disintegrant, a binder, and a lubricant.

3. The pharmaceutical composition according to claim 2, wherein the diluent comprises at least one selected from microcrystalline cellulose, anhydrous calcium hydrophosphate, and mannitol; preferably, the diluent is microcrystalline cellulose; and/or
the disintegrant comprises at least one selected from croscarmellose sodium, crospovidone XL-10, sodium carboxymethylcellulose, low-substituted hydroxypropyl cellulose, and pregelatinized starch; preferably, the disintegrant is croscarmellose sodium; and/or
the binder comprises at least one selected from hydroxypropyl cellulose, povidone K30, hydroxypropyl methylcellulose, and starch; preferably, the binder is hydroxypropyl cellulose; and/or
the lubricant comprises at least one selected from magnesium stearate, sodium stearyl fumarate, stearic acid, and talc; preferably, the lubricant is magnesium stearate; and/or
the glidant comprises a glidant selected from colloidal silicon dioxide.

4. The pharmaceutical composition according to any one of claims 1-3, further comprising an external lubricant.

5. The pharmaceutical composition according to any one of claims 2-4, wherein the content of the compound SHEN26 is 15 wt% to 70 wt% or 50 wt% to 60 wt%, based on the total mass of the pharmaceutical composition; and/or
the content of the diluent is 20 wt% to 70 wt% or 30 wt% to 40 wt%, based on the total mass of the pharmaceutical composition; and/or
the content of the disintegrant is 1 wt% to 10 wt% or 2 wt% to 4 wt%, based on the total mass of the pharmaceutical composition; and/or
the content of the binder is 1 wt% to 10 wt% or 4 wt% to 6 wt%, based on the total mass of the pharmaceutical composition; and/or
the content of the lubricant is 0.1 wt% to 5 wt% or 0.5 wt% to 1 wt%, based on the total mass of the pharmaceutical composition; and/or
the content of the external lubricant is 0.5 wt% to 5 wt% or 0.5 wt% to 1.5 wt%, based on the total mass of the pharmaceutical composition.

6. The pharmaceutical composition according to any one of claims 1-5, wherein the pharmaceutically acceptable excipient comprises a diluent, a disintegrant, a binder, a lubricant, or an external lubricant; the diluent is microcrystalline cellulose, the disintegrant is croscarmellose sodium, the binder is hydroxypropyl cellulose, and the lubricant is magnesium stearate; based on the total mass of the pharmaceutical composition, the content of the compound SHEN26 is 50 wt% to 60 wt%, the content of the diluent is 30 wt% to 40 wt%, the content of the disintegrant is 2 wt% to 4 wt%, the content of the binder is 4 wt% to 6 wt%, the content of the lubricant is 0.5 wt% to 1 wt%, and the content of the external lubricant is 0.5 wt% to 1.5 wt%.

7. The pharmaceutical composition according to any one of claims 1-6, wherein the dosage form of the composition is a solid oral dosage form.

8. The pharmaceutical composition according to claim 7, wherein the solid oral dosage form comprises a solid oral dosage form selected from a tablet, a granule, or a capsule.

9. The pharmaceutical composition according to any one of claims 1-8, wherein the specification of the pharmaceutical composition is 10 mg to 500 mg, or 50 mg, or 200 mg.

10. Use of the pharmaceutical composition according to any one of claims 1-9 in the preparation of a product for preventing, alleviating or treating a coronavirus infection, or replication or propagation of a homologous variant virus thereof, and a cytopathic effect generated therefrom.

11. The use according to claim 10, wherein the infection comprises fever, cough, sore throat, pneumonia, acute respiratory infection, severe acute respiratory infection, hypoxic respiratory failure and acute respiratory distress syndrome, sepsis, or septic shock.

12. Use of the pharmaceutical composition according to any one of claims 1-9 in the preparation of a product for detecting a coronavirus or a homologous variant virus thereof.

13. The use according to any one of claims 10-12, wherein the coronavirus comprises: MHV-A59, HCoV-229E, HCoV-OC43, HCoV-NL63, HCoV-HKU1, SARS-CoV, MERS-CoV, SARS-CoV-2, a mouse hepatitis virus, a feline infectious peritonitis virus, a canine coronavirus, a bovine coronavirus, an avian infectious bronchitis virus, or a porcine coronavirus; preferably, the SARS-CoV-2 comprises a mutant strain or a non-mutant strain of SARS-CoV-2; more preferably, the mutant strain of SARS-CoV-2 comprises SARS-CoV-2 mutant strain B.1, SARS-CoV-2 mutant strain B.1.351, SARS-CoV-2 mutant strain B.1.617.2, SARS-CoV-2 mutant strain C.37, SARS-CoV-2 mutant strain P.1 lineage, SARS-CoV-2 mutant strain B.1.525, SARS-CoV-2 mutant strain B.1.427, or SARS-CoV-2 mutant strain B.1.429.

14. The use according to any one of claims 10-13, wherein the pharmaceutical composition is suitable for use in humans or animals; and/or the animal comprises bovine, equine, ovine, porcine, canine, feline, rodent, primate, avian, or piscine animal.

15. A method for preparing the pharmaceutical composition according to any one of claims 1-9, comprising: mixing the compound SHEN26 and the pharmaceutically acceptable excipient, performing dry granulation, adding an external lubricant, mixing, and tableting or capsule filling or packaging to give the composition; or
mixing the compound SHEN26 and the pharmaceutically acceptable excipient, performing wet granulation, grinding, drying, dry grinding, adding an external lubricant, mixing, and tableting or capsule filling or packaging to give the composition; or
grinding or pulverizing the compound SHEN26, mixing the compound and the pharmaceutically acceptable excipient, performing dry granulation, adding an external lubricant, mixing, and tableting or capsule filling or packaging to give the composition; or
grinding or pulverizing the compound SHEN26, mixing the compound and the pharmaceutically acceptable excipient, performing wet granulation, grinding, drying, dry grinding, adding an external lubricant, mixing, and tableting or capsule filling or packaging to give the composition.
